# EUROPÄISCHE PATENTSCHRIFT

(11) **EP 3 388 090 B1**
(45) Veröffentlichungstag und Bekanntmachung des Hinweises auf die Patenterteilung: **06.11.2019**
(21) Anmeldenummer: 17166481.6
(22) Anmeldetag: 13.04.2017
(51) Int. Cl.: A61L 27/30, A61L 27/50

(54) **DOPPELBESCHICHTUNG FÜR EINE IMPLANTATKOMPONENTE MIT EINER REIBSCHLUSSVERBINDUNG**
DOUBLE LAYER COATING FOR AN IMPLANT COMPONENT WITH A FRICTIONAL ENGAGEMENT CONNECTION
REVÊTEMENT DOUBLE POUR UN COMPOSANT D'IMPLANT COMPRENANT UNE LIAISON PAR FRICTION

(43) Veröffentlichungstag der Anmeldung: 17.10.2018
(73) Patentinhaber: Waldemar Link GmbH & Co. KG, 22339 Hamburg (DE)
(72) Erfinder: Helmut D. LINK, 22397 Hamburg (DE); Richard, CSASZAR, 23795 Bad Segeberg (DE)
(74) Vertreter: Hoffmann Eitle

(56) Entgegenhaltungen:
- DE-A1-102014 206 151
- DE-U1-202012 104 253
- Helmut D Link: "Sonderausgabe Oktober 2013", , 1. Oktober 2013 (2013-10-01), Seiten 1-9, XP055414432, Gefunden im Internet: URL:https://www.linkorthopaedics.com/filea dmin/media/02_fuer_den_arzt/06_Direct_LINK /pdf_directLINK/DirectLINK_02-2013_dt.pdf [gefunden am 2017-10-11]

## Beschreibung

### GEBIET DER ERFINDUNG

Diese Erfindung betrifft eine Implantatkomponente mit einem männlichen und/oder weiblichen Reibverbindungsabschnitt, ein Verfahren zur Beschichtung einer Implantatkomponente und eine Implantatkomponente, die durch dieses Verfahren beschichtet wird.

### STAND DER TECHNIK

Bei Implantaten kommen aus unterschiedlichen Gründen Beschichtungen zum Einsatz. So können Beschichtungen eingesetzt werden, um eine bessere Integration des Implantats in Körpergewebe zu ermöglichen. Ein weiterer Grund ist eine Vorbeugung gegen Unverträglichkeiten oder allergische Reaktionen bei Patienten, die auf ein Implantat angewiesen sind, jedoch auf bestimmte Legierungskomponenten dieses Implantats hypersensitiv reagieren. Bei diesen Patienten ist es darum wichtig, gegen einen Kontakt mit diesen Legierungskomponenten vorzubeugen, um eine erfolgreiche Versorgung mit dem Implantat zu erreichen.

Hypersensitivitäten sind unter anderem bei Kobalt-Chrom-Legierungen bekannt. Um dennoch eine Implantation zu ermöglichen, können Implantate, die solche Legierungen aufweisen, mit einer Beschichtung aus Titan-Niob-Nitrid (TiNbN) oder Zirkonnitrid (ZrN) beschichtet werden, sodass der Implantationsort vor Kobalt- und Chrom-Ionen abgeschirmt wird. Allerdings sind diese Beschichtungen extrem hart, was sich negativ auf die Stabilität von Reibschlussverbindungen, insbesondere von Konusverbindungen, auswirken kann.

Reibschlussverbindungen werden häufig bei modular aufgebauten Implantaten, wie z. B. Gelenkimplantaten, eingesetzt und ermöglichen eine Verbindung über einen Reibschluss. Dieser wird durch ein Zusammenstecken der Konusverbindung und die dadurch hervorgerufene elastische Vorspannung erzeugt, die auf die Kontaktflächen der Reibschlussverbindung wirkt. Befindet sich auf einer oder beiden Kontaktflächen eine der zuvor erwähnten relativ harten Beschichtungen, so wird die Erzeugung der für die Verbindung notwendigen Vorspannung erschwert. Zudem kann durch eine Wechselwirkung zwischen der harten Beschichtung und der im Verhältnis dazu weicheren Legierung des Implantats eine Beschädigung der Beschichtung hervorgerufen werden. Folglich ist der Einsatz derartiger harter Beschichtungen im Bereich der Konusverbindung mit Schwierigkeiten verbunden.

Aus diesem Grund kommen weichere Beschichtungen zum Einsatz, welche ebenfalls die beiden Metalle Titan und Niob aufweisen können. Sowohl Titan als auch Niob sind für ihre Biokompatibilität bekannt. Im Gegensatz zu der harten TiNbN-Beschichtung werden diese Metalle jedoch nicht in einem Nitrid vereinigt, sondern als TiNb-Legierung aufgebracht. Der Vorteil dieser Beschichtung liegt insbesondere darin, dass sie einen im Vergleich zu Nitridbeschichtungen niedrigeren Elastizitätsmodul aufweist und somit eine feste Reibschlussverbindung, wie zum Beispiel die oben beschriebene Konusverbindung, ermöglicht. Eine solche Beschichtung wird zum Beispiel in der DE 10 2014 206 151 A1 offenbart.

Allerdings ist diese Beschichtung aufgrund ihrer niedrigeren Härte anfälliger für mechanische verursachte Beschädigungen, sodass die Handhabung des Implantats vor und während der Implantation eine höhere Sorgfalt erforderlich macht. Ansonsten besteht die Gefahr, dass bei einer Beschädigung dieser weicheren Beschichtungen Lücken in dieser entstehen, durch welche Legierungskomponenten entweichen und oben genannte Unverträglichkeiten oder allergische Reaktionen verursachen können.

Ähnlich hierzu offenbart die DE 20 2012 104 253 U1 eine erste und eine zweite Verbindungsfläche einer Endoprothese, die mit einer Tantal-Beschichtung überzogen sind.

### ZUSAMMENFASSUNG DER ERFINDUNG

Aufgabe der Erfindung war es somit, eine Beschichtung für eine Implantatkomponente bereitzustellen, die sowohl vor einem Absondern von Legierungsbestandteilen schützt, die eine Unverträglichkeit oder allergische Reaktion bei einem Patienten auslösen können, als auch eine problemlose Reibschlussverbindung ermöglicht.

Diese Aufgabe wird durch eine beschichtete Implantatkomponente nach Anspruch 1 gelöst. Die Implantatkomponente wird dabei bevorzugt mit dem Verfahren nach Anspruch 12 beschichtet. Die abhängigen Ansprüche definieren darüber hinaus weitere bevorzugte Ausführungsformen der Erfindung.

Wenn im folgenden auf das Material der Implantatkomponente Bezug genommen wird, ist hierunter das Material der unbeschichteten Implantatkomponente zu verstehen. Zudem wird die Implantatkomponente der vorliegenden Erfindung in einen Reibschlussverbindungsabschnitt und einen Funktionsabschnitt unterteilt. Der Funktionsabschnitt ist insbesondere ein Oberflächenabschnitt der Implantatkomponente, der aufgrund seiner Funktion oder zur Herstellung derselben stärker mechanisch beansprucht wird und somit eine oberflächliche Abnutzung wahrscheinlicher ist.

Des weiteren wird darauf hingewiesen, dass die im folgenden gewählten Bezeichnungen der Beschichtungen als erste Beschichtung und zweite Beschichtung willkürlich ist und nicht beabsichtigt ist, damit eine Reihenfolge anzudeuten, in der die Beschichtungen auf die Implantatkomponente aufgebracht werden bzw. auf dieser angeordnet sind.

Eine erfindungsgemäße Implantatkomponente weist einen männlichen und/oder weiblichen Reibschlussverbindungsabschnitt und einen Funktionsabschnitt auf. Der Reibschlussverbindungsabschnitt weist eine erste Beschichtung und der Funktionsabschnitt weist eine zweite Beschichtung auf. Zwischen den Beschichtungen ist zumindest ein Übergangsabschnitt mit der ersten und der zweiten Beschichtung vorgesehen. Die erste Beschichtung weist einen geringeren Elastizitätsmodul als die zweite Beschichtung auf.

Bei der erfindungsgemäßen Implantatkomponente ermöglicht die erste Beschichtung den zuverlässigen Einsatz eines Reibschlusses für eine Verbindung mit einer weiteren Implantatkomponente. Es wird angenommen, dass dies unter anderem an dem geringeren Elastizitätsmodul dieser Beschichtung liegt. Hierfür weist zumindest ein Teil des Reibschlussverbindungsabschnitts nicht die zweite Beschichtung und bevorzugt nur die erste Beschichtung auf.

Bei der Handhabung vor und während der Implantation, wie auch im implantierten Zustand, besteht jedoch die Gefahr einer Beschädigung der nachgiebigeren Beschichtung. Dabei kann es zu Abschabungen beim Einsetzen der Implantatkomponente und damit zu einer Durchlässigkeit für Legierungskomponenten kommen. Auch ist mittlerweile bekannt, dass bei Patienten in sehr seltenen Fällen eine Unverträglichkeit gegen Verbindungen auftreten kann, die selbst bei einer freiliegenden Titanoberfläche entstehen. Diese Unverträglichkeit wird insbesondere durch die aus der schnellen Passivierung von Titan hervorgehenden Verbindungen erklärt.

Um gegen einen direkten Kontakt zwischen dem Material der unbeschichteten Implantatkomponente und dem Körpergewebe vorzubeugen, ist zudem die zweite Beschichtung vorgesehen, mit der zumindest ein Teil des Funktionsabschnitts versehen ist. Die zweite Beschichtung kann damit in diesem Abschnitt das Körpergewebe hermetisch von dem Material der Implantatkomponente abschirmen, ohne dabei jedoch die Stabilität und Haltbarkeit der Reibschlussverbindung negativ zu beeinflussen.

Die Implantatkomponente weist den Übergangsabschnitt mit der ersten und der zweiten Beschichtung auf. Dieser Abschnitt kann in einem Teil des Reibschlussverbindungsabschnitts und/oder in zumindest einem Teil des Funktionsabschnitts vorgesehen sein.

Für den Fall, dass zumindest ein Teil des Übergangsabschnitts in dem Reibschlussverbindungsabschnitt angeordnet ist, ist dieser bevorzugt in einem Teil von diesem vorgesehen, der bei einem Reibschluss mit einer weiteren Implantatkomponente nicht mit dieser in Kontakt kommt. Mit anderen Worten muss nicht notwendigerweise der gesamte Reibschlussverbindungsabschnitt für eine Verbindung mit einer weiteren Implantatkomponente vorgesehen sein, sondern der Reibschlussverbindungsabschnitt kann bei einer Verbindung mit einer weiteren Implantatkomponente einen Kontaktteil und einen freiliegenden Teil aufweisen.

Weiterhin ist es möglich, den Übergangsabschnitt mit der ersten und zweiten Beschichtung im Wesentlichen in dem gesamten Funktionsabschnitt vorzusehen. Bei einer solchen Ausführung weist die beschichtete Implantatkomponente folglich einen Bereich auf, in dem keine zweite Beschichtung vorgesehen ist. Hingegen erstreckt sich die erste Beschichtung über die gesamte Implantatkomponente.

Insgesamt wirkt die zweite Beschichtung der vorliegenden Erfindung einer Unverträglichkeit von Legierungskomponenten der Implantatkomponente entgegen, da sie durch ihren höheren E-Modul und ihre höhere Härte unempfindlicher gegenüber Schäden ist und so gegen eine Freisetzung möglicherweise schädlicher Legierungskomponenten vorbeugt. Gleichzeitig ist durch die erste Beschichtung eine feste Reibschlussverbindung der Implantatkomponente mit einer anderen Implantatkomponente möglich.

Was für den geringeren Elastizitätsmodul der ersten Beschichtung gegenüber der zweiten Beschichtung zutrifft, gilt somit vorzugsweise auch für die Beziehung der Materialhärte dieser beiden Beschichtungen, d. h. die zweite Beschichtung weist eine höhere Härte als die erste Beschichtung auf. Folglich ist die zweite Beschichtung resistenter gegen Abrieb oder Beschädigung als die erste Beschichtung.

Bei einer besonders bevorzugten Ausführungsform ist der Funktionsabschnitt ein Schaftabschnitt zur Verankerung der Implantatkomponente an Knochengewebe oder eine Gelenkgleitfläche.

Für beide funktionellen Bereiche einer Implantatkomponente ist die zweite Beschichtung von Vorteil, um zuverlässig gegen einen Kontakt von möglicherweise schädlichen Legierungskomponenten mit dem Patientengewebe vorzubeugen. So ist im Fall einer rauen oder porösen Oberfläche, die insbesondere bei dem Schaftabschnitt einwachsender Implantatkomponenten vorgesehen ist, eine höhere Widerstandsfähigkeit der Beschichtung sinnvoll, um die Gefahr ihrer Verletzung zum Beispiel durch Abschabung bei der Implantaton zu senken.

Ähnliches gilt für den Fall einer Implantatkomponente mit einer Gelenkgleitfläche. Bei dieser ist es von Vorteil, den Abrieb so weit wie möglich einzuschränken, der durch die tägliche Bewegung des Patienten entsteht und langfristig zu einer Freilegung des Materials der Implantatkomponente führen kann.

Bei einer bevorzugten Ausführungsform weist die erste Beschichtung TiNb und/oder Zirkon auf oder besteht daraus.

Eine Beschichtung, die TiNb und/oder Zirkon aufweist, hat sich als besonders biokompatibel erwiesen. Dies ist insbesondere der Fall, wenn die Beschichtung aus Titan-Niob und/oder Zirkon besteht. Mit anderen Worten sind durch eine solche Beschichtung keine Unverträglichkeiten zu erwarten. Zudem weist diese Beschichtung eine Elastizität auf, die ausreicht, um mit dem Basismaterial der Implantatkomponente eine zuverlässige und stabile Reibschlussverbindung zu einer weiteren Implantatkomponente bereitzustellen.

Bei einer weiteren bevorzugten Ausführungsform ist die zweite Beschichtung eine Nitrid-Beschichtung. Vorzugsweise weist die Nitridbeschichtung dabei TiNbN und/oder ZrN auf oder besteht noch bevorzugter daraus.

Nitridbeschichtungen haben den Vorteil, dass sie sehr hart sind (> 2000 HV) und so einer mechanischer Verletzung einen erheblichen Widerstand entgegensetzen. Dies ist insbesondere während der Implantation von Vorteil, bei dem eine Gefahr eines Abrieb durch Operationswerkzeug wie auch durch das Knochengewebe des Patienten besteht. Genauso vorteilhaft ist der Einsatz einer solchen Beschichtung auf Gelenkgleitflächen, um deren Abnutzung so weit wie möglich entgegen zu wirken. Zudem haben sich sowohl TiNbN als auch ZrN für einen Einsatz in Kontakt mit Körpergewebe bewährt.

Bei einer weiteren bevorzugten Ausführungsform ist der Übergangsabschnitt zwischen der ersten und zweiten Beschichtung im Bereich des Funktionsabschnitts vorgesehen.

Diese Ausführungsform hat den Vorteil, dass sie die zweite Beschichtung vollständig von dem Bereich einer Reibschlussverbindung fernhält. Dies ist vor allem für die weit verbreiteten modularen Implantatsysteme vorteilhaft, da bei diesen eine Vielzahl unterschiedlicher Implantatkomponenten miteinander gekoppelt werden können, die nicht immer auf gleicher Höhe der Reibschlussverbindung anliegen.

Bei einer bevorzugten Ausführungsform ist die zweite Beschichtung im Bereich des Übergangsabschnitts auf der ersten Beschichtung angeordnet.

Diese Anordnung der Beschichtungen im Übergangsabschnitt hat insbesondere den Vorteil, dass die zweite Beschichtung als härteste Schicht bei der fertig beschichteten Implantatkomponente außen angeordnet ist und so sowohl gegen eine Verletzung des Materials der Implantatkomponente als auch gegen eine Verletzung der ersten Beschichtung vorgebeugt wird.

Bei einer weiteren bevorzugten Ausführungsform ist die erste Beschichtung im Bereich des Übergangsabschnitts auf der zweiten Beschichtung angeordnet.

Bei einer solchen Anordnung der Beschichtungen schützt die zweite Beschichtung weiterhin die Oberfläche der unbeschichteten Implantatkomponente. Der wesentliche Vorteil, d. h. dass keine möglicherweise problematische Legierungskomponente in das Gewebe des Patienten gelangen kann, bleibt somit erhalten. Hinzu kommt, dass auch im Fall einer Beschädigung der ersten Beschichtung keine negativen Effekte zu erwarten sind, da die erste Beschichtung wie auch die zweite Beschichtung biokompatible sind.

Ein weiterer Vorteil der Beschichtungsanordnung entsprechend dieser Ausführungsform liegt darin, dass die Herstellung der Beschichtungen vereinfacht wird. Genauer gesagt erfolgt zunächst eine Beschichtung mit dem härteren Beschichtungsmaterial, gefolgt durch eine Beschichtung mit dem weicheren Beschichtungsmaterial. Infolgedessen ist es einfacher, während der Beschichtung mit der ersten Beschichtung eine Beschädigung der zweiten, aufgrund ihrer Materialeigenschaften widerstandsfähigeren Beschichtung zu vermeiden.

Bei einer weiteren bevorzugten Ausführungsform weist die Implantatkomponente eine Kobalt-Chrom-Legierung auf oder besteht aus dieser.

Kobalt-Chrom-Legierungen haben aufgrund ihrer Materialeigenschaften vielfältige Verwendung bei Implantaten gefunden. So zeichnen sich diese Legierungen vor allem durch eine hohe Festigkeit aus, wodurch ein Versagen der Implantate durch die üblicherweise im implantierten Zustand auftretenden zyklischen Belastungen vermieden wird.

Bei einer weiteren Ausführungsform beträgt die Dicke der ersten Beschichtung mindestens 3 µm, bevorzugt mindestens 6 µm und maximal 12 µm und bevorzugt maximal 10 µm.

Das Festlegen der Beschichtungsdicke der ersten Beschichtung in diesen Bereichen stellt zum einen eine flächendeckende Beschichtung, insbesondere von stark strukturierten Implantatoberflächen, sicher und beugt gleichzeitig einer einfachen mechanischen Verletzung vor, welche die erste Beschichtung durchdringt.

Bei einer weiteren Ausführungsform beträgt die Dicke der zweiten Beschichtung mindestens 3 µm, bevorzugt mindestens 6 µm und maximal 10 µm und bevorzugt maximal 8 µm.

Auch für die zweite Beschichtung gelten die im Zusammenhang mit der ersten Beschichtung genannten Vorteile. Allerdings kann die maximale Dicke aufgrund der höheren Widerstandsfähigkeit der zweiten Beschichtung gegen mechanische Beschädigung geringer sein.

Bei einer weiteren Ausführungsform ist die Implantatkomponente eine Gelenkimplantatkomponente.

Gelenkimplantatkomponenten sind im Allgemeinen relativ große Implantate, die zudem für einen Einwuchs von Knochengewebe in die Oberfläche der Implantatkomponente ausgeführt sein können. Folglich kommt es insbesondere bei diesem Implantattyp zu einem großflächigen und engen Kontakt zwischen der Implantatkomponente und Knochengewebe. Gerade hier kann damit einer sich entwickelnden Überempfindlichkeit eines Patienten vorgebeugt werden.

Ähnliches gilt für die Gleitfläche einer Gelenkimplantatkomponente, wie zum Beispiel bei einem Hüftkopf, die zumindest indirekt über die Gelenkflüssigkeit mit dem Patientengewebe in Kontakt steht. Dementsprechend ist es auch bei der Gleitfläche von Vorteil, wenn das Material der Implantatkomponente abgeschirmt werden kann.

Zudem stellt die vorliegende Erfindung ein Verfahren zum Beschichten einer Implantatkomponente mit einem männlichen und/oder weiblichen Reibschlussverbindungsabschnitt und einem Funktionsabschnitt bereit. Das Verfahren umfasst die Schritte eines Bereitstellens der Implantatkomponente, einer Maskierung von zumindest einem Teil der Oberfläche des Reibschlussverbindungsabschnitts, eines Aufbringens einer zweiten Beschichtung, eines Entfernens der Maskierung des Reibschlussverbindungsabschnitts und eines Aufbringens einer ersten Beschichtung, wobei die erste Beschichtung einen geringeren Elastizitätsmodul als die zweite Beschichtung aufweist.

Eine mit diesem Verfahren hergestellte Implantatkomponente weist dementsprechend die oben genannten Vorteile auf, die durch eine Kombination der ersten und zweiten Beschichtung erreicht werden. Dabei ist der Schritt einer Maskierung von zumindest einem Teil der Oberfläche des Reibschlussverbindungsabschnitts vorgesehen, damit die beschichtete Implantatkomponente in zumindest dem oben bereits erwähnten vorbestimmten Kontaktbereich des Reibschlussverbindungsabschnitts nicht die zweite Beschichtung aufweist.

Folglich kann bei einer Möglichkeit zur Durchführung des erfindungsgemäßen Verfahrens zunächst in der nachfolgenden Reihenfolge zumindest ein Teil der Oberfläche des Reibschlussverbindungsabschnitts maskiert werden, eine zweite Beschichtung aufgebracht werden, die Maskierung des Reibschlussverbindungsabschnitts entfernt werden und schließlich eine erste Beschichtung aufgebracht werden.

Bei einer anderen Möglichkeit zur Durchführung des erfindungsgemäßen Verfahrens kann in der folgenden Reihenfolge zunächst eine erste Beschichtung aufgebracht werden. Dann wird zumindest ein Teil der Oberfläche des Reibschlussverbindungsabschnitts maskiert, bevor eine zweite Beschichtung auf die Implantatkomponente aufgebracht wird. Danach wird die Maske wieder entfernt.

Vorzugsweise werden die erste und zweite Beschichtung ohne eine weitere dazwischenliegende Beschichtung direkt auf das Material der Implantatkomponente aufgebracht.

Bei einer Ausführungsform des Verfahrens wird das Ausführen der Maskierung von zumindest einem Teil der Oberfläche des Reibschlussverbindungsabschnitts und einer Fixierung der Implantatkomponente gleichzeitig, d. h. in demselben Schritt ausgeführt.

Mit anderen Worten wird bei dieser Ausführungsform der Teil der Oberfläche des Reibschlussverbindungsabschnitts dadurch maskiert, dass er für das Aufbringen der zweiten Beschichtung gehalten wird und die Halterung dabei gleichzeitig eine Maske ausbildet. Auf diese Weise ist das Maskieren zeitsparend und zudem automatisch durchführbar.

Bei einer weiteren Ausführungsform des Verfahrens wird die erste und/oder zweite Beschichtung durch PVD, insbesondere durch Arc-PVD aufgebracht.

Diese Beschichtungsverfahren haben insbesondere den Vorteil, dass sie eine Erzeugung einer relativ großen Schichtdicke in einem Arbeitsgang ermöglichen. Insbesondere ist es möglich, die bevorzugten Schichtdicken in einem einzigen Arbeitsgang herzustellen. Als Ergebnis wird mit diesen Verfahren eine besondere homogene Beschichtung erzeugt, da sie aus einer einzigen und nicht aus mehreren Lagen besteht.

Weiterhin stellt die vorliegende Erfindung eine Implantatkomponente bereit, die durch eine Ausführungsform des zuvor beschriebenen Verfahrens beschichtet wird.

### AUSFÜHRLICHE BESCHREIBUNG BEVORZUGTER AUSFÜHRUNGSFORMEN

Bei einer erfindungsgemäßen Implantatkomponente werden insbesondere auf das Material der Implantatkomponente eine erste und eine zweite Beschichtung aufgebracht. Dabei weist die erste Beschichtung einen geringeren Elastizitätsmodul auf als die zweite Beschichtung. Die Beschichtungen sind so ausgewählt, dass sie im implantierten Zustand der Implantatkomponente keine überempfindlichen Reaktionen mit umliegendem Körpergewebe hervorrufen. Beispielsweise kann als erste Beschichtung TiNb und/oder Zr verwendet werden. Als zweite Beschichtung kommt vorzugsweise TiNbN und/oder ZrN zum Einsatz. Weiterhin werden vorzugsweise als erste und zweite Beschichtung die Kombination Zr/ZrN oder TiNb/TiNbN gewählt.

Die erste Beschichtung mit einem geringeren Elastizitätsmodul ist dabei vorgesehen, in dem vorbestimmten Bereich einer Reibschlussverbindung aufgebracht zu werden. Eine häufig im Gebiet der vorliegenden Erfindung verwendete Reibschlussverbindung ist zum Beispiel eine Konusverbindung. Diese kommt insbesondere bei modular aufgebauten Gelenkimplantaten zum Einsatz, um zum Beispiel Implantatkomponenten untereinander sowie mit einem Gelenkkörper zu verbinden.

Der geringere Elastizitätsmodul der ersten Beschichtung ist bevorzugt so gewählt, dass er näher an dem Elastizitätsmodul des Materials der Implantatkomponente liegt als der Elastizitätsmodul der zweiten Beschichtung. Auf diese Weise kann eine zuverlässige und stabile Reibschlussverbindung zwischen der beschichteten Implantatkomponente und einer weiteren Implantatkomponente erzeugt werden.

Zudem weist die zweite Beschichtung vorzugsweise eine höhere Härte als die erste Beschichtung auf. Hiermit wird bei dem Funktionsabschnitt einer Beschädigung und Abrieb vorgebeugt, die bei Implantation oder Verwendung der Implantatkomponente auftreten können. Diese höhere Härte liegt dabei vorzugsweise in einem Bereich von 2000 HV bis 3000 HV. Eine solche Härte kann zum Beispiel durch eine Nitridbeschichtung erreicht werden. Im Gegensatz zu der ersten Beschichtung wird die zweite Beschichtung also vorzugsweise in einem Nitrid vereinigt.

Die erste Beschichtung ist in dem Bereich des Reibschlussverbindungsabschnitts vorgesehen, der bei einer vorbestimmten Verbindung mit einer weiteren Implantatkomponente in Kontakt ist. Durch diesen verbindungstypisch sehr engen Kontakt ist der Bereich der ersten Beschichtung in dem Kontaktbereich abgedeckt. Folglich kann zumindest bei einer geringfügigen Beschädigung der ersten Beschichtung einem Kontakt zwischen Legierungsbestandteilen des Implantatkomponentenmaterials und Körpergewebe eines Patienten vorgebeugt werden.

Die erste Beschichtung weist keine in einem Nitrid vereinigten Legierungskomponenten auf. Vorzugsweise weist die Beschichtung TiNb und/oder Zr auf. Noch bevorzugter besteht die erste Beschichtung aus TiNb und/oder Zr.

Darüber hinaus befindet sich die erste Beschichtung zudem in einem Übergangsabschnitt, der in dem Reibschlussverbindungsabschnitt und/oder dem Funktionsabschnitt angeordnet ist. In diesem Übergangsabschnitt ist entweder die erste Beschichtung in Richtung vom Inneren des Implantats nach außen auf der zweiten Beschichtung oder die zweite Beschichtung auf der ersten Beschichtung angeordnet.

Bevorzugte Beschichtungskombinationen sind Zr als erste Beschichtung und ZrN als zweite Beschichtung oder TiNb als erste Beschichtung und TiNbN als zweite Beschichtung. Genauso ist es allerdings möglich, die Beschichtungen in den Paarungen Zr und TiNbN oder TiNb und ZrN zu verwenden.

Wie oben bereits ausgeführt, wird aus Sicht der Herstellung der beschichteten Implantatkomponente eine Anordnung der ersten Beschichtung auf der zweiten Beschichtung bevorzugt. So hat sich herausgestellt, dass bei dieser Reihenfolge eine fehlerfreie Beschichtung der Implantatkomponente wesentlich einfacher herzustellen ist als umgekehrt. Wie oben bereits erwähnt, wird dabei im Rahmen der Beschichtung bevorzugt ein PVD-Verfahren und insbesondere Arc-PVD eingesetzt.

Vorzugsweise befindet sich der Übergangsabschnitt ausschließlich im Funktionsabschnitt, also dem Teil der Implantatkomponente, der im implantierten Zustand mit keiner weiteren Implantatkomponente in Kontakt steht. Dies hat den Vorteil, dass insbesondere bei modular aufgebauten Implantatsystemen im Verbindungsbereich ein Kontakt mit der zweiten Beschichtung ausgeschlossen werden kann.

Beide Beschichtungen weisen bevorzugt die jeweils oben definierten Schichtdicken auf. Diese Dicken sind insbesondere ausgewählt, um auch unstetige Bereiche, wie zum Beispiel Kanten, zuverlässig zu beschichten und somit einem Austritt ungewünschter Legierungskomponenten entgegenzuwirken. Dies trifft insbesondere für Oberflächentopographien zu, die für einen Knocheneinwuchs eingerichtet sind. Solche Oberflächentopographien zeichnen sich im Allgemeinen durch eine höhere Rauheit sowie möglicherweise eine vorhandene Porosität aus.

### Beispiel 1: Aufbringen der zweiten Beschichtung auf die erste Beschichtung

Bei diesem Beispiel wird zunächst die erste Beschichtung und dann die zweite Beschichtung auf die Oberfläche einer bereitgestellten Implantatkomponente aufgebracht. Vor dem Aufbringen kann zumindest ein Teil des Funktionsabschnitts maskiert sein. Das Ausmaß der Maskierung hängt dabei von der Position und Erstreckung des Übergangsbereichs ab.

Befindet sich der Übergangsbereich zum Beispiel nur in dem Reibschlussverbindungsabschnitt, so ist zumindest der gesamte Funktionsabschnitt und möglicherweise ein Teil des Reibschlussverbindungsabschnitts zu maskieren.

Erstreckt sich der Übergangsbereich hingegen in den Funktionsabschnitt oder ist nur in diesem angeordnet, wird dementsprechend nur ein Teil des Funktionsabschnitts maskiert. Weiterhin ist es bei einer derartigen Anordnung der beiden Beschichtungen auch möglich, vor dem Aufbringen der ersten Beschichtung überhaupt keine Maske vorzusehen und folglich die gesamte Oberfläche der Implantatkomponente zu beschichten. Jedoch kann auch hier möglicherweise zum Halten der Implantatkomponente zumindest eine Stelle auf der Implantatkomponente "maskiert" sein, d. h aufgrund eines Kontakts mit einer Halterung während der Beschichtung bedeckt sein. Dies vereinfacht die Herstellung der Beschichtung zusätzlich und ist somit kostengünstiger.

Wie auch bei den nachfolgenden Beispielen gilt allgemein für die erste Beschichtung, dass eine Maskierung der Implantatkomponente nicht vollständig hermetisch sein muss, wohingegen die Maskierung vor dem Aufbringen der zweiten Beschichtung zumindest bei der für einen Reibschluss vorgesehenen Fläche hermetisch abdeckt sein sollte bzw. muss.

Als Nächstes erfolgt bei diesem Beispiel ein Aufbringen der ersten Beschichtung. Nach dem Aufbringen der ersten Beschichtung wird die genannte Maske, falls vorhanden, wieder entfernt.

Anschließend erfolgt ein weiterer Maskierungsschritt, bei dem zumindest ein Teil der Oberfläche des Reibschlussverbindungsabschnitts maskiert wird, d. h. zumindest der Teil, der für einen Kontakt mit einer weiteren Implantatkomponente vorgesehen ist. Wie oben beschrieben erfolgt diese Maskierung vorzugsweise mit der Halterung, die zum Halten der Implantatkomponente während des Beschichtungsprozesses mit der zweiten Beschichtung verwendet wird. Auch diese Maske erstreckt sich entsprechend der Anordnung des Übergangsabschnitts zumindest über den Teil der Oberfläche des Reibschlussverbindungsabschnitts, der für einen Kontakt mit einer weiteren Implantatkomponente zum Herstellen einer Reibschlussverbindung vorgesehen ist.

Ist die Maskierung abgeschlossen, erfolgt schließlich eine Beschichtung der Implantatkomponente mit der zweiten Beschichtung.

### Beispiel 2: Aufbringen der ersten Beschichtung auf der zweiten Beschichtung

Bei dieser Anordnung der zwei Beschichtungen aufeinander und auf der vorzugsweise unbeschichteten Implantatkomponente erfolgt zunächst eine Maskierung von zumindest dem Teil der Oberfläche des Reibschlussverbindungsabschnitts, der für einen Kontakt mit einer weiteren Implantatkomponente vorgesehen ist, um zwischen den beiden Implantatkomponenten eine Reibschlussverbindung zu erzeugen. Auch hier kann die Maskierung einfach durch die Halterung erfolgen, mit der die Implantatkomponente für den Beschichtungsprozess mit der zweiten Beschichtung gehalten wird. Einmal maskiert, erfolgt anschließend die Beschichtung mit der zweiten Beschichtung und danach eine Entfernung der Maske. Im Fall der Halterung erfolgt das Entfernen der Maske dementsprechend einfach durch Herausnahme der Implantatkomponente aus der Halterung.

Nach dem Beschichtungsvorgang mit der zweiten Beschichtung kann die Implantatkomponente erneut für die Beschichtung mit der ersten Beschichtung maskiert werden. Dabei beschränkt sich die Beschichtung in so einem Fall, wie oben bereits beschrieben, auf einen Bereich, der von der Erstreckung des Übergangsbereichs über die Implantatkomponente abhängig ist.

Auch bei diesem Beispiel ist es zudem möglich, wie auch schon bei dem vorgenannten Beispiel, auf eine Maskierung zu verzichten und stattdessen die erste Beschichtung im Wesentlichen auf der gesamten Oberfläche der zu beschichtenden Implantatkomponente aufzubringen. Im Wesentlichen bedeutet auch hier, dass bei dem Beschichtungsvorgang mit der ersten Beschichtung durch die Halterung trotzdem mindestens eine Stelle auf der Oberfläche der Implantatkomponente "maskiert" sein kann.

In jedem Fall wird durch die Beschichtung der Implantatkomponente bei der vorliegenden Erfindung einer Überempfindlichkeit oder allergischen Reaktion durch einen Kontakt mit Körpergewebe des Patienten vorgebeugt.

## Patentansprüche

1. Implantatkomponente mit einem männlichen und/oder weiblichen Reibschlussverbindungsabschnitt und einem Funktionsabschnitt, wobei der Reibschlussverbindungsabschnitt eine erste Beschichtung und der Funktionsabschnitt eine zweite Beschichtung aufweist, zwischen den Beschichtungen zumindest ein Übergangsabschnitt mit der ersten und der zweiten Beschichtung vorgesehen ist und die erste Beschichtung einen geringeren Elastizitätsmodul als die zweite Beschichtung aufweist.

2. Implantatkomponente nach Anspruch 1, bei welcher der Funktionsabschnitt ein Schaftabschnitt zur Verankerung der Implantatkomponente an Knochengewebe oder eine Gelenkgleitfläche ist.

3. Implantatkomponente nach Anspruch 1 oder 2, bei der die erste Beschichtung Titan-Niob und/oder Zirkon aufweist oder daraus besteht.

4. Implantatkomponente nach einem der Ansprüche 1 bis 3, bei der die zweite Beschichtung eine Nitridbeschichtung ist.

5. Implantatkomponente nach Anspruch 4, bei der die Nitridbeschichtung Titan-Niob-Nitrid und/oder Zirkonnitrid aufweist.

6. Implantatkomponente nach einem der vorstehenden Ansprüche, bei welcher der Übergangsabschnitt zwischen der ersten und zweiten Beschichtung im Bereich des Funktionsabschnitts vorgesehen ist.

7. Implantatkomponente nach einem der vorstehenden Ansprüche, bei der die zweite Beschichtung im Bereich des Übergangsabschnitts auf der ersten Beschichtung angeordnet ist.

8. Implantatkomponente nach einem der Ansprüche 1 bis 6, bei der die erste Beschichtung im Bereich des Übergangsabschnitts auf der zweiten Beschichtung angeordnet ist.

9. Implantatkomponente nach einem der vorstehenden Ansprüche, wobei die Implantatkomponente eine Kobalt-Chrom-Legierung aufweist oder aus dieser besteht.

10. Implantatkomponente nach einem der vorstehenden Ansprüche, bei der die Dicke der ersten Beschichtung mindestens 3 µm, bevorzugt mindestens 6 µm und maximal 12 µm und bevorzugt maximal 10 µm beträgt.

11. Implantatkomponente nach einem der vorstehenden Ansprüche, bei der die Dicke der zweiten Beschichtung mindestens 3 µm, bevorzugt mindestens 6 µm und maximal 10 µm und bevorzugt maximal 8 µm beträgt.

12. Verfahren zum Beschichten einer Implantatkomponente mit einem männlichen und/oder weiblichen Reibschlussverbindungsabschnitt und einem Funktionsabschnitt, wobei das Verfahren die Schritte umfasst:
- Bereitstellen der Implantatkomponente;
- Maskierung von zumindest einem Teil der Oberfläche des Reibschlussverbindungsabschnitts;
- Aufbringen einer zweiten Beschichtung;
- Entfernen der Maskierung des Reibschlussverbindungsabschnitts;
- Aufbringen einer ersten Beschichtung;
wobei die erste Beschichtung einen geringeren Elastizitätsmodul als die zweite Beschichtung aufweist.

13. Verfahren nach Anspruch 12, bei dem das Ausführen der Maskierung von zumindest einem Teil der Oberfläche des Reibschlussverbindungsabschnitts und eine Fixierung der Implantatkomponente gleichzeitig ausgeführt werden.

14. Verfahren nach einem der Ansprüche 12 bis 13, bei dem die erste und/oder zweite Beschichtung durch PVD, insbesondere durch Arc-PVD aufgebracht wird.

## Claims

1. Implant component having a male and/or female frictional connection portion and a functional portion, wherein the frictional connection portion has a first coating and the functional portion has a second coating, at least one transition portion with the first coating and the second coating is provided between the coatings, and the first coating has a lower elastic modulus than the second coating.

2. Implant component according to Claim 1, in which the functional portion is a shaft portion for anchoring the implant component to bone tissue or a sliding surface of a joint.

3. Implant component according to Claim 1 or 2, in which the first coating has or is composed of titanium-niobium and/or zirconium.

4. Implant component according to one of Claims 1 to 3, in which the second coating is a nitride coating.

5. Implant component according to Claim 4, in which the nitride coating has titanium-niobium-nitride and/or zirconium nitride.

6. Implant component according to one of the preceding claims, in which the transition portion between the first and second coating is provided in the region of the functional portion.

7. Implant component according to one of the preceding claims, in which the second coating is arranged on the first coating in the region of the transition portion.

8. Implant component according to one of Claims 1 to 6, in which the first coating is arranged on the second coating in the region of the transition portion.

9. Implant component according to one of the preceding claims, wherein the implant component has or is composed of a cobalt-chromium alloy.

10. Implant component according to one of the preceding claims, in which the thickness of the first coating is at least 3 µm, preferably at least 6 µm and at most 12 µm and preferably at most 10 µm.

11. Implant component according to one of the preceding claims, in which the thickness of the second coating is at least 3 µm, preferably at least 6 µm and at most 10 µm and preferably at most 8 µm.

12. Method for coating an implant component having a male and/or female frictional connection portion and a functional portion, said method comprising the steps of:
- making available the implant component;
- masking at least part of the surface of the frictional connection portion;
- applying a second coating;
- removing the masking of the frictional connection component;
- applying a first coating;
wherein the first coating has a lower elastic modulus than the second coating.

13. Method according to Claim 12, in which the masking of at least part of the surface of the frictional connection portion and a fixing of the implant component are carried out simultaneously.

14. Method according to either of Claims 12 and 13, in which the first and/or second coating is applied by PVD, in particular by arc PVD.

## Revendications

1. Composant d'implant comprenant une portion de liaison par engagement par friction mâle et/ou femelle et une portion fonctionnelle, la portion de liaison par engagement par friction présentant un premier revêtement et la portion fonctionnelle présentant un deuxième revêtement, au moins une portion de transition avec le premier et le deuxième revêtement étant prévue entre les revêtements et le premier revêtement présentant un module d'élasticité plus faible que le deuxième revêtement.

2. Composant d'implant selon la revendication 1, dans lequel la portion fonctionnelle est une portion de tige pour l'ancrage du composant d'implant à un tissu osseux ou à une surface de glissement d'articulation.

3. Composant d'implant selon la revendication 1 ou 2, dans lequel le premier revêtement présente du titane-niobium et/ou du zirconium ou se compose de ceux-ci.

4. Composant d'implant selon l'une quelconque des revendications 1 à 3, dans lequel le deuxième revêtement est un revêtement de nitrure.

5. Composant d'implant selon la revendication 4, dans lequel le revêtement de nitrure présente du nitrure de titane-niobium et/ou du nitrure de zirconium.

6. Composant d'implant selon l'une quelconque des revendications précédentes, dans lequel la portion de transition entre le premier et le deuxième revêtement est prévue dans la région de la portion fonctionnelle.

7. Composant d'implant selon l'une quelconque des revendications précédentes, dans lequel le deuxième revêtement est disposé dans la région de la portion de transition sur le premier revêtement.

8. Composant d'implant selon l'une quelconque des revendications 1 à 6, dans lequel le premier revêtement est disposé dans la région de la portion de transition sur le deuxième revêtement.

9. Composant d'implant selon l'une quelconque des revendications précédentes, dans lequel le composant d'implant présente un alliage de cobalt-chrome ou se compose de celui-ci.

10. Composant d'implant selon l'une quelconque des revendications précédentes, dans lequel l'épaisseur du premier revêtement vaut au moins 3 µm, de préférence au moins 6 µm et au maximum 12 µm et de préférence au maximum 10 µm.

11. Composant d'implant selon l'une quelconque des revendications précédentes, dans lequel l'épaisseur du deuxième revêtement vaut au moins 3 µm, de préférence au moins 6 µm et au maximum 10 µm et de préférence au maximum 8 µm.

12. Procédé de revêtement d'un composant d'implant comprenant une portion de liaison par engagement par friction mâle et/ou femelle et une portion fonctionnelle, le procédé comprenant les étapes suivantes :
- fourniture du composant d'implant ;
- masquage d'au moins une partie de la surface de la portion de liaison par engagement par friction ;
- application d'un deuxième revêtement ;
- enlèvement du masquage de la portion de liaison par engagement par friction ;
- application d'un premier revêtement ;
le premier revêtement présentant un plus faible module d'élasticité que le deuxième revêtement.

13. Procédé selon la revendication 12, dans lequel la réalisation du masquage d'au moins une partie de la surface de la portion de liaison par engagement par friction et une fixation du composant d'implant sont effectuées simultanément.

14. Procédé selon l'une quelconque des revendications 12 et 13, dans lequel le premier et/ou le deuxième revêtement sont appliqués par PVD, en particulier par arc-PVD.
